# EUROPEAN PATENT APPLICATION

(11) **EP 2 016 975 A1**
(43) Date of publication of application: **21.01.2009**
(21) Application number: 07118151.5
(22) Date of filing: 09.10.2007
(51) Int. Cl.: A61N 1/36

(54) **Method for restoring an ejaculatory failure**

(30) Priority: 12.07.2007 US 949251 P
(71) Applicant: Pelvipharm, 75016 Paris (FR)
(72) Inventor: Bernabe, Jacques, 92120 Montrouge (FR); Borgdorff, Aren, 92100 Boulogne Billancourt (FR); Giuliano, François, 92210 Saint Cloud (FR)
(74) Representative: Cabinet Plasseraud

(57) **Abstract**

The present invention relates to a method for eliciting ejaculation in a male individual, comprising delivering one or more stimulation pulses to lumbar spinothalamic (LSt) cells.

## Description

### FIELD OF THE INVENTION

The present invention relates to methods for eliciting ejaculation in a male individual possibly suffering from an ejaculation failure.

### BACKGROUND OF THE INVENTION

Sexual performance in humans involves many functions: in males, these functions mainly are erection, ejaculation and orgasm. A wide variety of medical and psychological problems may also interfere with one or more of these functions. Methods to treat these sexual dysfunctions are known in the art. For example, US patent 6,169,924 describes stimulation of the spinal cord to achieve orgasm, whereas to achieve erection, US2005/0222628 patent application describes stimulation of the pelvic nerve and US2005/0096709 patent application describes electrical stimulation of the prostate gland.

However, ejaculation is not the same as orgasm from a physiological perspective: in particular, in spinal cord injured patients ejaculation can be achieved without orgasm. Ejaculation comprises two distinct and successive phases: emission and expulsion. Emission involves transport of spermatozoa from the epidydimis along the vas deferens and their mixing with secretions from prostate and seminal vesicles SV (semen) before terminating as sperm in the prostatic urethra. Expulsion is the forceful expulsion of sperm from the urethra out of the urethral meatus and depends on the coordinated and rhythmic contraction of the striated perineal muscles, in particular the bulbospongiosus BS muscle.
Ejaculation is thus a complex mechanism, and the prior art failed in proposing solutions for eliciting simultaneously the whole process: US2005/0222628 patent application describes stimulation of the pelvic plexus nerves to achieve emission but fails to address the expulsion issue; US2005/0096709 patent application describes electrical stimulation of the prostate gland to achieve ejaculation, but electrical stimulation of the prostate gland only causes emission and not expulsion. Therefore, known treatments for ejaculation failure allow only the first phase of ejaculation i.e. emission but do not lead to a complete ejaculation with expulsion of the sperm.

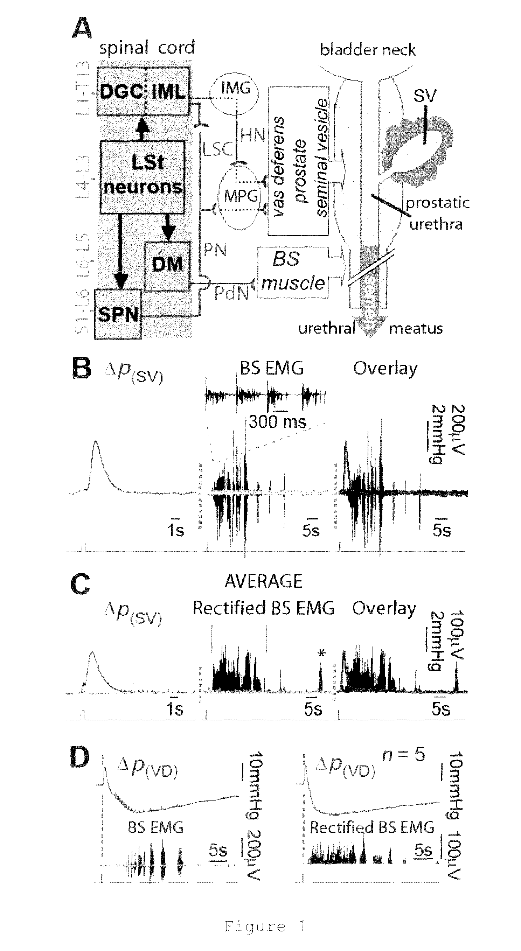

Recently, new data have been published providing a better comprehension of the mechanism of ejaculation. Ejaculation can occur in response to genital stimulation in humans and rats after complete lesion of the spinal cord above T10, evidencing that the spinal cord is still able to command and organize the peripheral events leading to ejaculation. In rats, LSt neurons in lamina VII and X of the spinal segment L3-L4 have been postulated to form a spinal generator for ejaculation (SGE) to coordinate the sympathetic, parasympathetic and somatic efferent activities (Truitt and Coolen, 2002, Science 297:1566). During copulation in rats, the expression of a marker for neuronal activity, c-Fos, increases in L3-L4 LSt neurons after ejaculation and not after mounts and intromissions (Truitt and Coolen, 2003, J Neurosci. 23:325) (Figure 1A).

Coolen et al (US2004/0152631) mention a method comprising the administration to an individual of a drug such as neurotransmitters, for example gamma-amino-butyric acid, or neuropeptides for example serotonin, galanin, somatostatin, which may interact with LSt cells. They suggest that this method would allow manipulation of the sensation of ejaculation; however, they do not prove that it could lead to the restoration of an ejaculation failure.
Nevertheless, LSt neurons still provide an interesting target for eliciting ejaculation, and , taking into account that chemical drugs may induce undesirable side effects, the Applicant focussed on alternatives means to medication, for eliciting ejaculation or restoring an ejaculation failure, such as for example anejaculation, which is a common ejaculatory dysfunction in spinal-cord-injured men.

### SUMMARY OF THE INVENTION

The invention relates to a method for eliciting ejaculation in a male individual, comprising delivering one or more stimulation pulses to lumbar spinothalamic (LSt) cells via a suitable device, in an effective amount to activate LSt cells for achieving expulsion of sperm. According to an embodiment, the device is implantable. Advantageously, the device is a medical device. The stimulation pulses may be all kinds of pulses delivered by an implantable device placed in the spinal cord between the lumbar spinothalamic L1 to L4 segments, more preferably in lamina VII and X in the area where lumbar spinothalamic cells are located or in close vicinity to them, and preferably are electric pulses delivered by electric means placed in said areas.

According to an embodiment, the individual suffers from an ejaculation failure.

According to another embodiment, the device delivers pulses having a pulse rate ranging from 2 to 450 pulses per second, preferably 100 to 400 pulses per second, and more preferably 200 to 300 pulses per second.

According to another embodiment, the device delivers pulses having a pulse width ranging from 0.5 to 450 milliseconds, preferably from 0.5 to 200 milliseconds, and more preferably from 0.5 to 100 millliseconds.

According to another embodiment, the device delivers pulses which amplitude ranges from 1 to 10 volts.

According to another embodiment, the device delivers pulses which intensity ranges from 1 µA to 3 mA, preferably from 10 µA to 1 mA and more preferably to 100 µA to 500 µA.

The invention also relates to the use of a device for eliciting ejaculation in a male individual, wherein said device delivers stimulation pulses to lumbar spinothalamic (LSt) cells, wherein the device is preferably placed in the area of lumbar spinothalamic L1 to L4 segments, more preferably in lamina VII and X where lumbar spinothalamic cells are located

### DETAILED DESCRIPTION OF THE DRAWINGS

**Figure 1**: Ejaculation-related events elicited by electrical microstimulation of LSt neurons.
   (**A**) Schematic representation of connections of LSt neurons with pelvi-perineal anatomical structures involved in rat ejaculation. DGC: dorsal gray commisure; IML: intermediolateral column; DM: dorso-medial part of Onuf's nucleus; SPN: sacral parasympathetic nucleus; HN: hypogastric nerve; LSC: lumbosacral paravertebral sympathetic chain; PN: pelvic nerve; PdN: pudendal nerve; IMG: intermesenteric ganglion; MPG: major pelvic ganglion; BS: bulbospongiosus, SV: seminal vesicle. Spinal level for each nucleus is indicated in gray.
   **(B)** Simultaneous recording of Δ*p*_{(SV)} (dark gray) and BS EMG (black) elicited by LSt neuron microstimulation in an anesthetized rat. Stimulation protocol: 300 ms of 0.5 ms biphasic current pulses repeated at 200 Hz (60 pulses). Stimulation amplitude: ≥ 3 times the Δ*p*_{(SV)} response threshold. Light gray traces: background activity. The overlay (right) displays the sequential activation of SV and BS muscle after LSt neuron microstimulation. Inset, middle panel: evoked BS EMG on an expanded timescale shows 4 bursts within the BS EMG response, demonstrating the regular rhythmic bursting pattern.
   **(C)** Averaged response for recordings from 7 animals (experimental protocol and color codes as in B). BS EMG is shown rectified and 200 Hz low-pass filtered. Vertical lines: onset of BS EMG activity and time when 95% of BS EMG activity has occurred. Asterisk: late burst of BS EMG activity after LSt neuron microstimulation.
   **(D)** Simultaneous recording of Δ*p*_{(VD)} (dark gray) and BS EMG (black) elicited by LSt neuron microstimulation [stimulation protocol as in B]. Left panel: example experiment. Right panel: averaged response for recordings from 5 animals. Light gray traces: background activity.

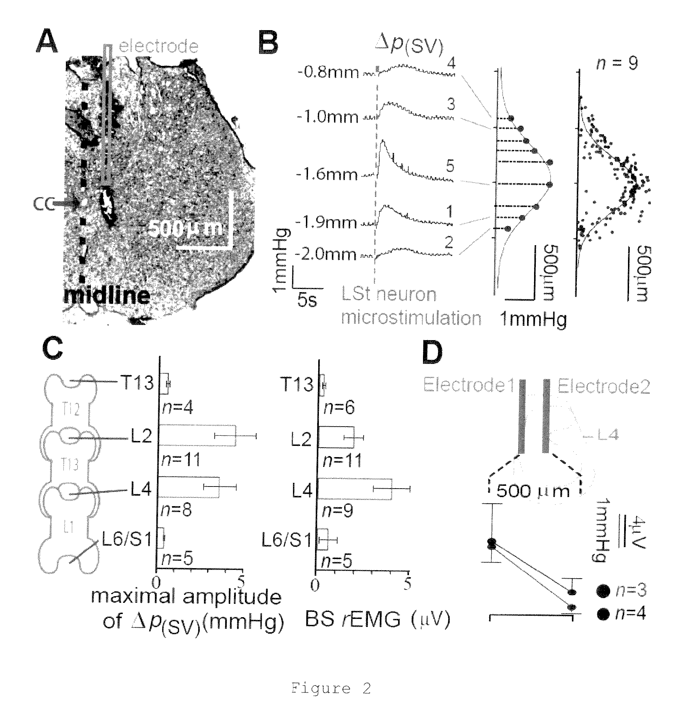

**Figure 2****:** The spinal location where microstimulation evokes ejaculation-related events corresponds to the LSt neuron area.
   **(A)** Strong current injections through the stimulation electrode at the end of the experiment marked the spinal location from which ejaculation and ejaculation-related events could be evoked (30 µm spinal cord section at L4, cc = central canal).
   (**B**) Left: Δ*p*_{(SV)} response (dark gray) in a single rat for various electrode depths, i.e. vertical distance from the dorsal surface of the spinal cord at L4 level. Stimulation time and stimulation sequence are shown.
      Middle: corresponding maximal amplitude of Δ*p*_{(SV)} (dots) vs. electrode depth (vertical axis) . The Gaussian function (black) used for data fitting peaks at -1537 µm from the dorsal surface of the spinal cord.
      Right: summary data for the maximal amplitude of Δ*p*_{(SV)} and electrode depth (*n* = 9) with mean Gaussian fit (black). For each animal, data was normalized by the Δ*p*_{(SV)} maximal amplitude and the peak position of the Gaussian.
   (**C**) Microstimulation applied at L2 and L4 evoked the highest maximal amplitude of Δ*p*_{(SV)} (left) and BS *r*EMG (right). Animal numbers indicated for spinal levels.
   (**D**) When placing two electrodes spaced 500 µm laterally at L4, a significantly larger maximal amplitude of Δ*p*_{(SV)} (*P <* 0.0032, red) and BS *r*EMG (*P* < 0.027, black) were evoked nearest to the spinal cord midline.

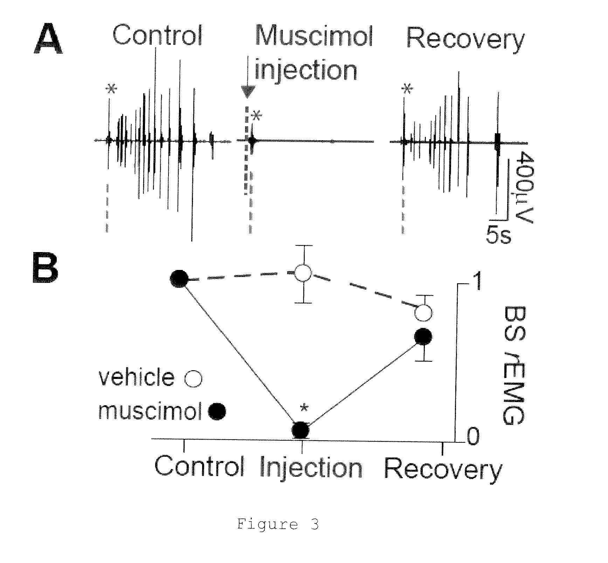

**Figure 3****.** L4 spinal microstimulation activates LSt neurons lying in the immediate vicinity of the stimulation electrode.
   (**A**) Example experiment. A first (left, black) intraspinal microstimulation of LSt neurons applied at L4 eliciting BS EMG activity (control). Injection of ~200 nl of a 50 µM solution of the GABA_{A}-receptor agonist muscimol in the vicinity of the electrode tip (middle trace, arrow) immediately followed by a second identical microstimulation abolished BS EMG activity. A third (right trace) microstimulation, ~20 minutes after muscimol injection (recovery), showed partial response recovery. This suggests that microstimulation activates neurons in the spinal area where LSt neurons are located and not axon fibers crossing the area. Asterisks represent stimulation artifacts.
   **(B)** Summary data for muscimol (*n* = 5) and vehicle (*n* = 4) injections. Mean BS *r*EMG ± SEM was normalized to the control values. Asterisk: *P* < 10⁻⁵ as compared to the control response.

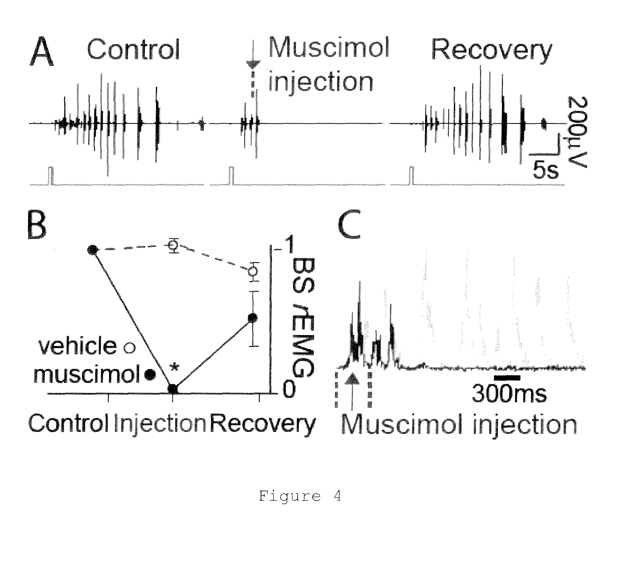

**Figure 4****.** LSt neuron activity is essential to maintain BS EMG rhythmic activity.
   **(A)** Example experiment. First (left, black) intraspinal microstimulation of LSt neurons applied at L4 eliciting BS EMG activity (control). A second (middle) identical microstimulation, followed 3.4 s later by injection of ~200 nl of a 50 µM solution of the GABA_{A}-receptor agonist muscimol (arrow) in the LSt neuron area adjacent to the electrode tip, completely interrupted the BS EMG activity. A third (right) microstimulation, ~20 minutes after muscimol injection (recovery), showed partial response recovery.
   **(B)** Summary data for muscimol (*n* = 5) and vehicle (*n* = 4) injections into LSt neuron area. Mean BS *r*EMG ± SEM, calculated for the interval 0.3-15.3 s after injection, was normalized towards the control values. Asterisk: *P* < 10⁻⁵ as compared to control response.
   **(C)** Muscimol injection (arrow; total duration 400 ms as indicated by dashed lines) rapidly turned off BS EMG activity. Dark gray trace: average of 5 animals. Data between animals normalized towards BS *r*EMG of the control response (light gray trace).

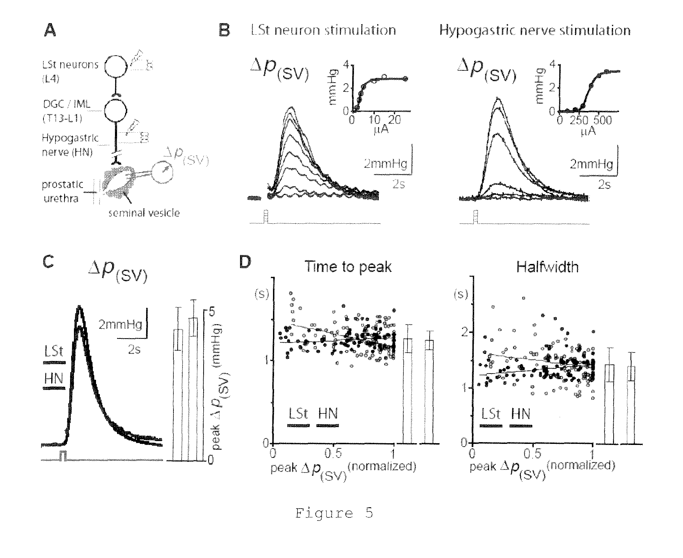

**Figure 5**. The timecourse of the SV contraction elicited by LSt neuron microstimulation is best explained by a short burst of hypogastic nerve (HN) activity.
   (**A**) Schematic representation of the experiment. LSt neurons project to preganglionic sympathetic neurons in the T13-L1 DGC / IML which in turn project to the SV via the HN. Brief and identical electrical stimulation patterns (60 pulses at 200 Hz, 300 ms train length) were applied to LSt neurons at L4 and right HN in different rats and the resulting SV contractions, recorded as the Δ*p*_{(SV)} response, compared.
   **(B)** Example experiments showing the Δ*p*_{(SV)} timecourse after LSt neuron (left) and HN (right) stimulation for different stimulation amplitudes. Insets: Δ*p*_{(SV)} peak amplitude vs. stimulation amplitude: the Δ*p*_{(SV)} peak amplitude reaches a maximum for the highest stimulation amplitudes.
   **(C)** Mean maximum Δ*p*_{(SV)} peak amplitude elicited by LSt-neuron-(*n* = 13, dark grey) and HN- (*n* = 11, black) stimulation.
   **(D)** Quantification of the Δ*p*_{(SV)} timecourse elicited by LSt neuron stimulation (*n* = 13, dark grey) and by HN stimulation *(n =* 11, black) for experiments as shown in (B) and (C). Left: time-to-peak. Right: half width. Each data point reflects a single Δ*p*_{(SV)} peak amplitude, normalized towards the maximum Δ*p*_{(SV)} peak amplitude in each individual experiment (3-8 values per experiment). Straight lines: data fit with a linear function. Bar graphs: mean value ± SEM.

From the close similarities in the *Δp*_{(SV)} timecourse (panels B, C and D) between LSt neuron stimulation and HN stimulation, we infer that brief LSt-neuron stimulation causes a short burst of activity in the DGC / IML preganglionic neurons which is conveyed by the HN to cause the observed SV contraction.

### DETAILED DESCRIPTION OF THE INVENTION

The present invention thus relates to a method for eliciting ejaculation in a male individual or for restoring an ejaculation failure in a male individual suffering therefrom, comprising delivering one or more stimulation pulses to lumbar spinothalamic (LSt) cells via a suitable device, in an effective amount to activate LSt cells for achieving expulsion of sperm. The present invention also relates to the use of a device for eliciting ejaculation in a male individual or for restoring an ejaculation failure in a male individual suffering therefrom, wherein said device delivers stimulation pulses to lumbar spinothalamic (LSt) cells.

One object of the present invention is to provide a method for eliciting ejaculation in a male individual, comprising delivering one or more stimulation pulses to lumbar spinothalamic (LSt) cells via a suitable device, in an effective amount to activate LSt cells for achieving expulsion of sperm.
In an embodiment of the invention, said device is implantable.
In a preferred embodiment of the invention, said stimulation pulses are electric pulses delivered by electric means placed in the area of lumbar spinothalamic L1 to L4 segments, preferably in the area of L2 to L4 segments.
In a preferred embodiment of the invention, said device is placed in lamina VII and X of lumbar spinothalamic L1 to L4 segments where LSt cells are located.

In the meaning of the present invention, a male individual refers to a male human being or a male animal, preferably a mammal. Preferably, a male individual means a man or a boy over 16. In a first embodiment, said male individual is suffering from an ejaculation failure. In a second embodiment, said male individual is not suffering from an ejaculation failure.

Restoring an ejaculation failure may be considered as a medical need and in the embodiment of the invention where the male individual of the invention necessitates a restoration of ejaculation functions, the device used in this invention may be considered as a medical device. Example of male individual necessitating a restoration of ejaculation functions are spinal-cord-injured men suffering from anejaculation.

However, according to another embodiment, this invention may also be useful for eliciting ejaculation in males which are not suffering from a medically-recognized deprivation/impairment of their ejaculation, and in this embodiment, the device of the invention shall be considered as a personal healthcare device.

In the meaning of this invention "ejaculation" comprises two distinct and successive phases: emission and expulsion of sperm.
The "effective amount" is to be determined by the person skilled in the art. In this context, a testing lead position may be tested to determine the effective amount of electrical stimulation. The testing lead is secured for trial screening of the individual's response to stimulation. A percutaneous extension is connected to the implanted lead and external percutaneous wires are connected. The individual then goes to a trial-screening period. In case the trial-screening period is ineffective, the lead may be repositioned. If the trial-screening period is positive, the device is internalized within the patient. According to an embodiment, "effective amount" is variable among male individuals but generally corresponds within a rate range of approximately 2 to 450 pulses per second, a pulse width range of approximately 0.5 to 450 milliseconds, and an amplitude range of approximately up to 10 volts.
According to a preferred embodiment, the device used in the invention is equipped with a controller operable by the individual or by the physician. The controller may be programmed so that the device delivers an arbitrarily limited number of stimulations of predetermined length to prevent overstimulation. The best program for each individual patient can be only determined by testing. These parameters include number of pulses grouped, voltage, rates and pulse duration. Programmable parameters are:
- intensity ranging preferably from 100µA to 500µA
- amplitude ranging preferably from 1 to 10 volts,
- pulse width ranging from 0.5 to 450 milliseconds, preferably 0.5 to 200 milliseconds, more preferably 0.5 to 100 milliseconds),
- pulse rate ranging from 2 to 450 pulses per second, preferably 100 to 400 pulses per second, and more preferably 200 to 300 pulses per second,
- mode (continuous or cycling), preferably the mode is cycling, and
- cycle (on/off), said cycle being inhibited for a period, such as every 1000 seconds.

Devices delivering stimulation pulses are well known in the art.

In an embodiment of the invention, the device used in this invention includes stimulating leads, connected to a power source that generates the electrical stimulation, and possibly an extension wire that conducts electrical stimulation from the power source to the electrode or lead. In the meaning of the invention, the term "lead" refers to a small conductor with a set of electrodes that delivers electrical stimulation. Examples of leads are monopolar leads, bipolar leads, or leads including any number of leads. The term "extension wire" refers to a small conductor that electrically connects the power source to the lead.
Examples of power sources are battery-powered stimulator with non invasive programmability, battery-powered rechargeable stimulator with non invasive programmability or radio-frequency system, which consists of an implanted receiver that detects radio-frequency signals through the skin from an external power source or transmitter.

According to an embodiment, the device used in the present invention comprises an electric power supply having more than one voltage source. According to an embodiment, the device delivers low voltage pulses.

In another embodiment, the medical implantable device may also comprise a processor to select and define the pulse sequence and command the power source, and a programmer which can be used to program the desirable stimulation sequence to the power source via telemetry. The programmer may comprise a physician programmer that allows the physician to define and upload a pulse sequence to the power source. Alternatively, the programmer may comprise a patient programmer which allows the patient to select the timing of therapy.
Using the programmer, the physician may specify the stimulation parameters, such as the voltage or the current amplitude, width, and rate of stimulation pulses.
The power source and the programmer may communicate via wireless communication using RF telemetry techniques known in the art. They may also communicate with each other using any of a variety of local wireless communication techniques, such as RF communication according to the 802.11 or Bluetooth specification sets, or other standard or proprietary telemetry protocols.

Advantageously, said leads are placed in the body in the area of lumbar spinothalamic L1 to L4 segments, in order to deliver electrical stimulation to LSt cells. More preferably, said device is placed in lamina VII and X of lumbar spinothalamic L1 to L4 segments or where Lst cells are located. Upon placement, electrical stimulation should be supplied in an effective amount to achieve sufficient activation of LSt cells and lead to ejaculation, i.e. emission and expulsion.

According to a preferred embodiment, the device used in this invention is totally implantable in the patient body. According to another embodiment, the device is partly implanted and includes both internal and external components. For example, only the means for delivering the pulses, such as for example leads, and a radio-frequency receiver, are internal, i.e. implanted within the body and the power source external, i.e. not implanted.

The present invention will be more fully understood in view of the following examples.

### EXAMPLES

### Materials and methods

### Animals and surgery

All procedures were in accordance with the European Communities Council Directives 86/609/EEC on the use of laboratory animals. Male adult sexually naïve Wistar rats (Janvier, Le Genest-St-Isle, France) of 275-325 grams, housed for at least 4 days in our animal facility before experimentation, were anesthetized with 1.2 mg/kg intraperitoneal urethane while body temperature was maintained at 37°C with a homeothermic blanket. Paw withdrawal and eye blink reflexes were largely suppressed. Custom-made bipolar steel wire electrodes (AS631, CoonerWire, CA, USA) were implanted into the exposed dorsal part of the right bulbospongiosus (BS) muscle. After suprapubic midline abdominal incision, a 1.1 mm diameter mineral oil-filled catheter was inserted into the lumen of the right seminal vesicle (SV) via its apex or the right vas deferens (VD) was cut and a 0.61 mm diameter catheter filled with isotonic salt solution inserted into the prostatic portion of the VD lumen. Then, the spine was exposed dorsally and fixed with a stereotaxic frame. Laminectomy was performed between vertebrae L1-T13 to expose L4 spinal level and the dura was carefully removed. To improve intraspinal access, we incubated the spinal cord for 20 minutes with 3 units/ill collagenase type VII from Clostridium histolyticum (Sigma-Aldrich Chimie, St.Quentin Fallavier, France). To target T12 to L6 spinal levels in Figure 2C, we performed 2-3 laminectomies on the same animal. Acute spinalization was performed through a complete spinal transection between T8 and T9 spinal levels one hour before microstimulation started. We performed bilateral sectioning of i) the hypogastric and pelvic nerves close to the major pelvic ganglion or iii) the dorsal nerves of the penis at the penile crus. A single experiment lasted 2 to 4 hours.

### Spinal Microstimulation

Monopolar spinal microstimulation was performed with a 'Formvar'-coated nichrome wire of 50 µm diameter (AM-Systems Inc. WA, USA). Typically, the electrode was positioned on the dorsal surface at L4 spinal level, adjacent to the right of the dorsal spinal artery and lowered vertically with a hydraulic microdrive (Trent-Wells, Coulterville, CA, USA) to ~1600 µm depth in correspondence with the stereotaxic coordinates for laminae VII and X (S2), taking as electrode depth the read-out of the microdrive. A reference electrode was placed in the vicinity of the tail. Electrical stimuli were delivered using a pulse generator (model-2100, AM-Systems Inc. WA, USA). Biphasic rectangular current pulses of 0.5 ms duration applied in short trains of 60 to100 pulses at 200 Hz for a total duration of 300 to 500 ms were applied. This stimulus was optimal without causing temporal overlap between stimulus and the SV/VD/BS muscle responses, according to preliminary experiments. The stimulation amplitude was set to ≥ 3 times the threshold for eliciting an SV, VD and/or BS response (15-100 µA). For each stimulation, the ejaculate was collected on a coverslip and directly put under the microscope (Olympus CH-2, Olympus SAS, France; magnification 40x) in order to detect and observe spermatozoa. Sometimes, but not always, we observed BS EMG activity during the time of microstimulation, often associated with hind leg movements. This activity, unrelated to the ejaculation response, persisted in the presence of the GABA_{A} agonist muscimol as compared to controls (58 ± 25%; *n* = 5, *P* = 0.07) (Fig. 2E asterisks) and therefore was probably related to the recruitment of long-distance in passage axonal fibers that cross the stimulation area. For repeated stimulations (Fig. 2, B to E and Fig. 3) we kept stimulation parameters constant and respected an interval of 200 s or 400 s between two stimulations. In order to minimize the influence of the observed use-dependent decrease in BS EMG activity, we kept stimulation numbers to a minimum (3 per electrode locations and 2/3 different electrode locations per animal for Fig. 2C) or we microstimulated different locations repeatedly in an intercalated fashion and averaged the response values (Fig. 2D).

### Verification of the lateral position of the spinal microstimulation electrode

At the end of the experiment, spinal cord tissue was lesioned with 2-3 repeats of 1-2 mA current injections through the electrode used in the LSt stimulation protocol. The animal was then perfused transcardiacally for 15 minutes with ~600 ml 4% paraformaldehyde, the spinal cord removed and sliced into 30 µm thick slices with a cryostat. The shortest distance between the centre of the lesion and the spinal cord midline was taken as the electrode lateral position.

### Hypogastic nerve (HN) stimulation

After suprapubic midline abdominal incision, the right HN was exposed, placed on a bipolar Ag-AgCl hook electrode close to the major pelvic ganglion and kept in a pool of paraffin oil. Electrical stimuli were applied to one leg of the electrode, the other serving as reference electrode. Stimulus trains were identical to those applied to the spinal cord, with current amplitudes ranging between 0.2-1 mA to evoke SV luminal pressure changes.

### BS muscle EMG and intraluminal SV/VD pressure change recording

EMG from the proximal part of the BS muscle (BS EMG), was recorded differentially, amplified and filtered (model-1700, AM-Systems Inc., USA; amplification 1000x, bandpass filter settings 0.1-1 kHz). To quantify SV contraction, luminal SV pressure change (Δ*p*_{(SV)}) was measured at the tip of the oil-filled tube (total length ~200 mm) with a pressure sensor (26PCAFG6G, Honeywell Inc., USA) connected to a bridge amplifier (TRN005, Kent Scientific Corp., UK; amplification 1000x or 2000x, 100 Hz lowpass filter). In preliminary experiments, we confirmed that Δ*p*_{(SV}) recorded with this technique closely related to *in situ* values measured simultaneously with a miniature pressure probe (SambaSensors SAB, Sweden), with only ~5% error in absolute values. To quantify VD contraction, luminal VD pressure change (Δ*p*_{(VD)}) was measured at the tip of the tube (total length ~300 mm, filled with isotonic salt solution) with a pressure sensor. Basal VD luminal pressure was increased to 37 ± 4 mmHg (*n* = 5) through continuous perfusion of the tube with isotonic salt solution at a rate of 2.25 µl*min⁻¹. This procedure aimed to prevent obstruction of the tube tip, but also explained the decrease in VD pressure after VD contraction as seen in Fig 1D, reflecting refilling of the VD with isotonic salt solution. Data was stored at 5 kHz sampling rate on a PC for later analysis.

### Local spinal drug application

For intraspinal drug delivery, a glass micropipette (100-150 µm tip diameter) with 1 µl calibration lines (Drummond Scientific Co., Broomall, PA, USA) was glued to the stimulation electrode with its opening at electrode tip level and slowly inserted into the LSt neuron area at the L4 level using stereotaxic coordinates (S2). The micropipette was filled with an isotonic salt solution (vehicle) with or without 50 µM muscimol (Sigma-Aldrich Chimie SARL, France). For drug delivery, pressure pulses (0.75 bar, three 20-80 ms pulses for a total duration of 400 ms) were applied to the backend of the pipette with a computer-controlled 'PicroSpritzer' (Intracell Ltd., UK) synchronized with the pulse generator. The total injection volume was 200-500 nl. Depending on the experiment, electrical microstimulation followed injection by 150 ms (Fig. 3) or preceded injection by 3.4 s (Fig. 4). A ~20-minute recovery period was respected before the next microstimulation took place.

### Data analysis

BS EMG, Δ*p*_{(SV)} and Δ*p*_{(VD)} recordings were analyzed using custom written routines in Elphy software (G. Sadoc, CNRS, Gif-sur-Yvette, France). Mean baseline values over 1 s before microstimulation were subtracted from each recording trace before analysis. For BS EMG quantification, EMG signals were rectified, 200 Hz lowpass filtered and the mean value was calculated between 1 and 25 s after the end of microstimulation. We called this the mean rectified BS EMG (BS *r*EMG). In Fig. 2C and Fig. 2D each BS *r*EMG value represents the average of 3 consecutive sweeps respectively. For BS EMG burst frequency calculation, the time interval between the start of the 1^{st} burst and the end of the 5^{th} burst were determined visually. For the Δ*p*_{(SV)} and Δ*p*_{(VD)} maximal amplitude we determined the maximum value for Δ*p*_{(SV)} and Δ*p*_{(VD)} between 0.5 and 4 s after the end of microstimulation. In Fig. 2C and Fig. 2D each Δ*p*_{(SV)} maximal amplitude represents the average of 4-7 consecutive sweeps. For the sampling of electrode locations along the dorso-ventral spinal axis (Fig. 2B), we randomly sampled 8-13 locations (minimum interval of 100 µm or 200µm) in each experiment. Data fitting was done in Excel (Microsoft Inc., USA) using a generalized reduced gradient (GRG2) algorithm. For graphical display, we removed stimulation artefacts from the EMG data, with the exception of Fig. 3A. Presented values are given as means ± standard error of the mean (SEM). To test statistical significance we used Student's *t*-test with a *P*-value < 0.05 considered significant.

### Results

Brief electrical microstimulation of LSt neurons evoked ejaculation, the expulsion of semen at the urethral meatus, in 17 out of 17 anesthetized adult rats. In 10 out of the 17 rats, motile spermatozoa were observed by optical bright field microscopy. In the other 7 animals, the ejaculate contained immotile or no spermatozoa.

To further characterize ejaculation elicited by LSt neuron microstimulation, we quantified three critical parameters of ejaculation: i) SV contraction was recorded via SV luminal pressure change (Δ*p*_{(SV)}), ii) BS muscle activity was recorded with a BS muscle electromyogram (BS EMG) and iii) VD contraction was recorded via VD luminal pressure change (Δ*p*_{(VD)}). After the application of 60-100 current pulses (200 Hz) in the LSt neuron area at L4, the SV luminal pressure immediately rose and fell, followed by prolonged rhythmic contractions of the BS muscle (Fig. 1B and C). Δ*p*_{(SV)} followed a smooth curve, reaching a maximum value of 4.05 ± 0.64 mmHg, 1.34 ± 0.08 s after the onset of LSt neuron microstimulation and with a half width of 1.24 ± 0.04 s (*n* = 12). SV contraction kinetics is likely the consequence of strong activation of the hypogastric nerves as a result of LSt stimulation (Fig. 5). BS EMG activity in the form of bursts (Fig. 1B, inset middle panel) started 3.2 ± 0.08 s after the onset of LSt neuron microstimulation. Furthermore, 95% of the BS EMG activity had occurred at 25 ± 2 s (*n* = 23). Occasional BS muscle contractions were observed even -50 s after the end of LSt neuron microstimulation (asterisk in Fig. 1C). The first 5 bursts of BS muscle activity occurred at a frequency of 2.4 ± 0.2 Hz (*n* = 23). Similar burst-like behavior in the BS EMG has been observed in copulating and anesthetized rats during ejaculation. In separate experiments we observed an increase in VD luminal pressure elicited by LSt neuron stimulation (Fig. 1D) . Δ*p*_{(VD)} reached a maximum value of 9.8 ± 0.91 mmHg, 0.66 ± 0.03 s after the onset of LSt neuron microstimulation (*n* = 5). The present data shows that brief LSt neuron stimulation suffices to sequentially activate the peripheral physiological events leading to emission and expulsion.

The spinal area from which we could successfully evoke ejaculation when applying microstimulation was then assessed. According to the read-out of the micromanipulator, the usual position of the stimulation electrode was at a depth of -1600 µm from the dorsal surface of the spinal cord at L4 and the lateral position was 210 ± 30 µm (*n* = 12) lateral from midline (Fig. 2A). We first sampled different electrode locations along the dorso-ventral axis at L4. The maximal amplitude of Δ*p*_{(SV)} varied systematically with electrode depth, following a bell-shaped depth profile (Fig. 2B) . By fitting the experimental data with a Gaussian function, the highest maximal amplitude of Δ*p*_{(SV)} was determined at a spinal depth of 1620 ± 35 µm and the half maximal amplitude at 550 ± 20 µm above or below this position (*n =* 11). This range of electrode locations, for which microstimulation evoked a maximal Δ*p*_{(SV)} response, corresponds to laminae VII and X, which match the region around the spinal midline where the LSt neurons are located. Along the rostro-caudal axis (Fig. 2C), the amplitude of the Δ*p*_{(SV)} response was maximal at L2 and L4. Almost negligible responses were observed rostrally (T13) and caudally (at the border between L6 and S1) (Fig. 2C, left panel). BS EMG responses showed the same pattern along the rostro-caudal axis (Fig. 2C, right panel). Bursting BS EMG activity (Fig.1B) was observed in 34 out of 40 trials (85%) (n = 20 animals) with microstimulation applied at L2 or L4 at a depth of 1500 µm and 1700 µm below the dorsal surface of the spinal cord. At T13 and L6-S1 the BS EMG bursting was only observed in 2 out of 22 trials (9%) (*n* = 11 animals). Lastly, for the medio-lateral axis we placed two electrodes at L4 spaced 500 µm apart (Fig. 2D). Keeping electrical stimulation conditions identical, the laterally located electrode yielded a significant decrease in i) the maximal amplitude of Δ*p*_{(SV)} (reduced to 10 ± 6 %, *n =* 3, *P* = 0.027) and ii) the mean rectified BS EMG response (BS *r*EMG) (reduced to 27 ± 6 %, *n* = 4, *P* = 0.0032) when compared to the medially located electrode. Altogether, we could evoke ejaculation or ejaculation-related events when applying electrical microstimulation within an area of ~0.5 mm diameter around the central canal at L2 and L4. This is in good agreement with the LSt neuron location around the central canal at L1-L5 in rats. We therefore conclude that we have stimulated LSt neurons in the present experiments.

We then verified that electrical microstimulation activated LSt neurons and not axon fibers in passage. We made use of two observations: i) GABA_{A} receptors are abundantly expressed on the cell body and proximal dendrites of lamina VII/X spinal neurons, but not on axon fibers, ii) GABA_{A}-receptor activation reduces neuron excitability. A small quantity of the GABA_{A}-receptor agonist muscimol was thus delivered in the LSt neuron area at L4, in the direct vicinity of the tip of the microstimulation electrode. Muscimol injection was immediately followed by electrical microstimulation of the LSt neurons (Fig. 3A). Under these conditions, BS EMG activity following microstimulation was greatly reduced to 6 ± 5% (*n* = 5, *P* < 10⁻⁵) of the control response (Fig. 3B). A direct block of the DM motoneurons activity by muscimol can be excluded as a likely explanation for our observations, because the distance between the muscimol injection site and the DM motoneurons exceeds 2 mm. These results confirm that ejaculation induced by microstimulation in the LSt neuron area is due to activation of neurons whose somata or proximal dendrites are confined to an area immediately surrounding the stimulation electrode.

LSt neurons connect to thalamic centers and may receive somatosensory and viscerosensory input from the periphery. If activated by LSt neuron stimulation, thalamic centers and/or peripheral sites could contribute to the ejaculation evoked by microstimulation. Because BS EMG activity occurred considerably after the end of the LSt neuron stimulation, this activity could be driven by other sources than LSt neurons. Therefore, we performed experiments in which we isolated the LSt neurons from thalamic or peripheral sites, through respectively acute T8-T9 spinalization (*n* = 4) or acute bilateral lesion of pelvic and hypogastric nerves (*n* = 5) or dorsal nerves of the penis (*n* = 6) prior to LSt neuron microstimulation. None of these interventions did alter the activation pattern of the evoked BS EMG response. By excluding thalamic and peripheral contributions we demonstrate that the ejaculatory motor pattern elicited by microstimulation applied in the LSt neuron area is generated by the spinal cord.

In a final step, we investigated whether LSt neuron activation generates the expulsion phase of ejaculation. First, we elicited rhythmic BS EMG activity by LSt neuron microstimulation. When this activity was well under way (i.e. 3.4 s after the end of LSt neuron microstimulation), we suppressed LSt neuron activity through the focal application of muscimol in the direct vicinity of the stimulation electrode (Fig. 4A). This turned off the rhythmic pattern of BS EMG activity to 2 ± 2% of the control response (Fig. 4B) (*n* = 5, *P* < 10⁻⁵). The BS EMG activity showed partial recovery, when evoked again -20 minutes later by LSt neuron microstimulation. Muscimol injection exerted its full suppressive effect within 160 ± 120 ms after muscimol injection (Fig. 4C). Because the blockade of LSt neuron activity abolishes BS EMG activity in midstream, we conclude that LSt neuron activation not only initiates ejaculation, but is also required to maintain the rhythmic BS EMG activity during the expulsion phase of ejaculation. This last experiment demonstrates that LSt neurons are the generator of ejaculation.

LSt neurons or neurons in their immediate vicinity activate the entire sequence of ejaculation in anesthetized male adult rats. We have provided direct evidence for the activation of the emission (SV and VD contractions) and expulsion (BS EMG activity) phases. The presence of motile spermatozoa in the expulsed semen indicates VD contractions to be propulsive and prostatic secretion, known to be crucial for sperm motility. The expulsion of semen at the urethral meatus also suggests that the bladder neck must be closed. Midcourse interruption of LSt neuron activity during the expulsion phase abolishes BS EMG activity immediately and reversibly. This clearly establishes that the SGE is located in the LSt neurons area. Accordingly, LSt neurons are the SGE or a crucial component of the SGE. The present experiments do not, however, tell whether all LSt neurons are part of the SGE or whether the generator extends beyond the LSt neuron area. Further investigations should include evidence for the rhythmogenic capacity of LSt neurons and detailed study of their pharmacology.

Given the comparable spinal organization for ejaculation in rats and humans our results could help to identify spinal pharmacological targets for the treatment of premature, delayed or absent ejaculation in man. These results also provide the rationale to investigate intraspinal stimulation for anejaculation, a common ejaculatory dysfunction in SCI patients, 90% of whom need medical assistance to procreate. Current fertilization methods require highly sophisticated interventions associated with increased risk for the female partner and infant. Intraspinal microstimulation in SCI patients through an implantable device could facilitate intravaginal ejaculation during sexual intercourse combined, when necessary, with erectile dysfunction treatment and thereby help these patients to impregnate their partner in a more physiological manner.

## Claims

1. Method for eliciting ejaculation in a male individual, comprising delivering one or more stimulation pulses to lumbar spinothalamic (LSt) cells via a suitable device, in an effective amount to activate LSt cells for achieving expulsion of sperm.

2. A method according to claim **1,** wherein said device is implantable.

3. A method according to anyone of claims **1** or **2,** wherein said stimulation pulses are electric pulses delivered by electric means placed in the area of lumbar spinothalamic L1 to L4 segments.

4. A method according to anyone of claims **1** to **3,** wherein said device is placed in lamina VII and X of lumbar spinothalamic L1 to L4 segments.

5. A method according to anyone of claims **1** to **4,** wherein the individual suffers from an ejaculation failure.

6. A method according to anyone of claims **1** to **5,** wherein the pulse rate ranges from 2 to 450 pulses per second.

7. A method according to anyone of claims **1** to **6,** wherein the pulse width ranges from 0.5 to 450 milliseconds.

8. A method according to anyone of claims **1** to **6,** wherein the pulse amplitude ranges from 1 to 10 volts.

9. Use of a device for eliciting ejaculation in a male individual, wherein said device delivers stimulation pulses to lumbar spinothalamic (LSt) cells.

10. Use according to claim **9,** wherein said stimulation pulses are electric pulses delivered via said device placed in the area of lumbar spinothalamic L1 to L4 segments.

11. Use according to anyone of claims **9** to **10,** wherein said device is placed in lamina VII and X of lumbar spinothalamic L1 to L4 segments.
